# EUROPEAN PATENT APPLICATION

(11) **EP 4 603 084 A1**
(43) Date of publication of application: **20.08.2025**
(21) Application number: 23890782.8
(22) Date of filing: 14.11.2023
(51) Int. Cl.: A61K 9/70, A61K 31/137, A61K 47/32, A61P 11/10, A61P 11/12

(54) **AMBROXOL ORAL DISSOLVING FILM COMPOSITION, PREPARATION METHOD THEREFOR, AND USE THEREOF**

(30) Priority: 14.11.2022 CN 202211420255
(71) Applicant: Shanghai Aurora Biotechnology Co., Ltd., Shanghai 201210 (CN); Taizhou Bocimed Pharmaceutical Co., Ltd., Taizhou, Jiangsu 225316 (CN)
(72) Inventor: YING, Shuhuan, Shanghai 201210 (CN); GUO, Zhen, Shanghai 201210 (CN); FU, Jun, Shanghai 201210 (CN); LU, Pengcheng, Shanghai 201210 (CN); LIU, Yuli, Shanghai 201210 (CN); WANG, Tingting, Shanghai 201210 (CN)
(74) Representative: Gille Hrabal Partnerschaftsgesellschaft mbB Patentanwälte
(86) International application number: PCT/CN2023/131591
(87) International publication number: WO 2024/104349

(57) **Abstract**

The present invention provides an ambroxol oral dissolving film composition, a preparation method therefor, and use thereof. The ambroxol oral dissolving film composition comprises an active drug, a film-forming material, and a taste masking agent. The active drug is 2-amino-3,5-dibromo-N-(trans-4-hydroxycyclohexyl)benzylamine and/or a pharmaceutically acceptable salt thereof, such as ambroxol hydrochloride. The ambroxol oral dissolving film composition provided by the present invention has the advantages of small thickness, good taste, stable properties, immediate dissolution in the mouth without drinking water, and high oral absorption speed. Moreover, the ambroxol oral dissolving film composition has a simple process, high drug-loading capacity, good drug content uniformity, and good marketization prospects.

## Description

The present application claims priority to the prior Patent Application No. 202211420255.4 entitled "AMBROXOL HYDROCHLORIDE ORAL DISSOLVING FILM COMPOSITION, PREPARATION METHOD THEREFOR, AND USE THEREOF" filed with the China National Intellectual Property Administration on November 14, 2022, which is incorporated herein by reference in its entirety.

### TECHNICAL FIELD

The present disclosure relates to an ambroxol hydrochloride oral soluble film composition, a method for preparing same, and use thereof.

### BACKGROUND

Ambroxol hydrochloride is an active metabolite of the expectorant bromhexine having lower toxicity and higher activity than bromhexine. Ambroxol hydrochloride is a mucolytic agent developed by Boehringer Ingelheim, Germany. It was first approved in Germany in the early 1980s and then was successively approved in many countries including France, Italy, Japan, Spain, etc. Ambroxol hydrochloride is one of the new generation of mucolytic agents. It is capable of improving expectoration, and possesses the functionality of promoting pulmonary surfactant, airway secretion, and cilium movement. Clinically, ambroxol hydrochloride is capable of regulating mucus and mucilage secretion, activating cilium swing, facilitating phlegm dilution, enhancing mucus outward transportation, and facilitating mucus discharge. It is also capable of promoting the synthesis of pulmonary surfactant to maintain the alveolar surface tension and ensure lung function indices, promoting the infiltration of antibiotics in tissues to increase the concentration and enhance their bactericidal effects. Moreover, it may serve as an anti-oxidant to reduce inflammatory mediator release and thereby alleviate inflammatory responses, and may be used in combination with bronchial spasmolytic substances to improve the therapeutic effects of the spasmolytic drugs. Therefore, clinically, the drug is widely used in acute or chronic respiratory tract diseases accompanied by abnormal secretion in respiratory tracts, particularly for the expectorant treatment of chronic bronchitis, the treatment of neonatal respiratory distress syndrome, and the adjuvant treatment of a lung surgery. It has the advantages of low toxicity, definite therapeutic effect, allowing combination therapy with antibiotics, and good synergistic effect, etc., and is one of the most common expectorants.

Currently, the supplier of the originator product provides dosage forms including injections, tablets, sustained-release capsules, and oral solutions in China. The drug has been approved in the following countries: Austria, Belgium, Bulgaria, Croatia, Cyprus, Czech Republic, Denmark, Estonia, France, Germany, Greece, Hungary, Iceland, Ireland, Italy, Latvia, Lithuania, Luxembourg, Malta, Norway, Poland, Portugal, Romania, Slovak, Slovenia, Spain, Sweden, and the Netherlands.

In addition, generic products such as injections, oral solutions, syrups, granules, tablets, capsules, sustained-release tablets, sustained-release capsules, orally disintegrating tablets, dispersible tablets, chewable tablets, etc., have been approved in China.

Ambroxol is popular among clinicians due to its significant expectorant effects, and its injection is widely used for nebulizer treatment (off-label) in patients with respiratory system diseases. However, actually, ambroxol injections are weakly acidic, and the nebulized ambroxol injection will also stimulate the airway, resulting in airway spasm and triggering dyspnea, and the adverse effect is more significant in COPD patients. In addition, in terms of either the diameter of the nebulized particles or the auxiliary materials (including preservatives), the injection prepared in normal saline may bring risks of pulmonary infection or inducing asthma exacerbation after being nebulized and inhaled. The new drug application (JXHS1700033) (reviewed with priority as a "pediatric drug") of ambroxol hydrochloride solution for inhalation submitted by Hanmi Pharm as a category 5.2 new drug was approved by the National Medical Products Administration in 2019, to fill the vacancy where existing dose forms are incapable of meeting clinical requirements in pediatric patients who need extensive ambroxol use.

In China, ambroxol oral formulations are all over-the-counter (OTC) products and are the popular ones among respiratory system drugs. They are used in a wide range of populations, especially infants and children. Also, instructions for ambroxol hydrochloride oral solutions clearly suggest that they are suitable for use in infants. However, there is much controversy over the use of such products in infants globally. Many institutions are concerned about the suitability of ambroxol as an expectorant in children aged 6 years or younger and believe that the benefits of such medications do not outweigh the risks in this population in this patient population.

The administration of normal tablets and sustained-release capsules of ambroxol requires water drinking and swallowing, and the patient compliance of these dosage forms is poor in some elders, children, and those with dysphagia. Therefore, the use of such products may be restricted by some certain conditions (e.g., the lack of potable water) or may have poor effects. Injections also have potential safety concerns and poor patient compliance. Although oral solutions have good taste, they possess the defects of a greater dose, difficulties in quantification, poor stability, etc., as well as higher requirements on production processes and on packaging and lower portability, thus bringing inconveniences and safety concerns to the administration in patients, carrying, transport, etc. Ambroxol hydrochloride is slightly soluble in water and has a bitter and tingling taste , and the bitterness and tingling sensation persist for a long time, which can not be masked by adding a solubilizer and a flavoring agent conventionally, resulting in poor patient compliance and particularly, disadvantages in administration in children. Patent No. CN102846581A discloses an ambroxol hydrochloride oral soluble film, which, however, as demonstrated by experiments, has poor mechanical strength, poor stability, slow disintegration speed, and a complex preparation process, and is thus disadvantageous to commercial production.

Therefore, the development of an ambroxol hydrochloride pharmaceutical formulation with good stability, good taste, and good patient compliance is urgently needed.

### SUMMARY

In order to overcome the defects including poor taste, poor stability, and/or poor patient compliance of ambroxol hydrochloride oral fast-soluble film in the prior art, the present disclosure provides an ambroxol hydrochloride oral soluble film composition, a method for preparing same, and use thereof. The ambroxol hydrochloride oral soluble film composition provided by the present disclosure has the advantages of thin thickness, good taste, stable properties, stable mechanical properties, immediate dissolution in the oral cavity without drinking water, and high oral absorption speed. Meanwhile, it has a simple process, a high drug-loading rate, good drug content uniformity, and good marketing prospects.

The present disclosure provides an ambroxol oral soluble film composition, comprising an active drug, a film-forming material, and a taste-masking agent, wherein the active drug comprises 2-amino-3,5-dibromo-N-(trans-4-hydroxycyclohexyl)benzylamine (also known as ambroxol) or a pharmaceutically acceptable salt thereof, e.g., 2-amino-3,5-dibromo-N-(trans-4-hydroxycyclohexyl)benzylamine hydrochloride (also known as ambroxol hydrochloride) of the formula I:

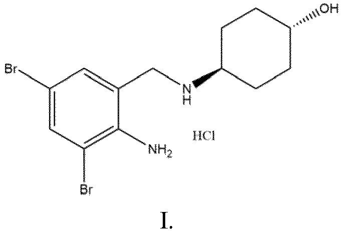

According to an embodiment of the present disclosure, the ambroxol oral soluble film composition described herein can be an ambroxol hydrochloride oral soluble film composition.

According to an embodiment of the present disclosure, preferably, the ambroxol oral soluble film composition (e.g., the ambroxol hydrochloride oral soluble film composition) described herein does not comprise a pH regulator.

According to an embodiment of the present disclosure, preferably, the ambroxol oral soluble film composition (e.g., the ambroxol hydrochloride oral soluble film composition) described herein does not comprise a free pH regulator. The free pH regulator refers to a pH regulator that does not form a salt with the active drug.

According to an embodiment of the present disclosure, the pH regulator includes, but is not limited to, a pharmaceutically acceptable acid known in the art for adjusting the pH, e.g., an organic acid, an inorganic acid, an organic base, and an inorganic base. The inorganic acid described above includes, e.g., hydrochloric acid, hydrobromic acid, sulfuric acid, nitric acid, phosphoric acid, etc.; the organic acid described above includes, e.g., acetic acid, propionic acid, glycolic acid, oxalic acid, maleic acid, malonic acid, succinic acid, fumaric acid, tartaric acid (citric acid), benzoic acid, mandelic acid, methanesulfonic acid, ethanesulfonic acid, toluenesulfonic acid, sulfosalicylic acid, formic acid, trifluoroacetic acid, etc.

According to an embodiment of the present disclosure, the ambroxol oral soluble film composition (e.g., the ambroxol hydrochloride oral soluble film composition) described herein does not comprise a basic pH regulator, e.g., a pharmaceutically acceptable base known in the art for adjusting the pH. According to an embodiment of the present disclosure, the active drug is present at a mass percentage of 1.00% to 40.00%, preferably 5.00% to 30.00%, e.g., 5.00%, 6.00%, 7.00%, 8.00%, 9.00%, 9.09%, 10.00%, 12.45%, 15.00%, 15.23%, 16.48%, 19.23%, 19.48%, 20.00%, 21.43%, 25.00%, or 30.00%, wherein the mass percentage refers to the percentage of the mass of the active drug relative to the total mass of the ambroxol oral soluble film composition (e.g., the ambroxol hydrochloride oral soluble film composition).

According to an embodiment of the present disclosure, the taste-masking agent is a substance for masking unpleasant tastes such as bitter taste, tingly taste, etc., and may be selected from a cation exchange resin, wherein the cation exchange resin is selected from one, two, or three of sodium polystyrene sulfonate, polacrilin potassium, and polacrilin resin.

Preferably, the cation exchange resin has a particle size D90 of less than 200 µm.

Preferably, the taste-masking agent is present at a mass percentage of 10.00% to 70.00%, more preferably 15.00% to 60.00%, e.g., 10.00%, 12.00%, 15.00%, 19.48%, 20.00%, 21.43%, 24.90%, 25.00%, 30.00%, 30.30%, 38.06%, 38.46%, 40.00%, 41.21%, 45.00%, 45.45%, 50.00%, 55.00%, or 60.00%, wherein the mass percentage refers to the percentage of the mass of the taste-masking agent relative to the total mass of the ambroxol oral soluble film composition (e.g., the ambroxol hydrochloride oral soluble film composition).

According to an embodiment of the present disclosure, the film-forming material is a carrier of the drug and is selected from one or more of xanthan gum, guar gum, pectin, gelatin, shellac, gum arabic, starch, dextrin, agar, sodium alginate, zein, hydroxypropyl methylcellulose, hydroxymethylcellulose, hydroxyethylcellulose, hydroxypropylcellulose, polyvinyl alcohol, pullulan, polyvinylpyrrolidone, polyethylene glycol, polyoxyethylene, acrylic acid copolymer, polylactic acid, and silicone rubber.

Preferably, the film-forming material is present at a mass percentage of 10.00% to 70.00%, preferably 15.00% to 60.00%, e.g., 10.00%, 12.00%, 14.29%, 15.00%, 20.00%, 25.00%, 27.47%, 30.00%, 30.30%, 30.45%, 35.00%, 37.34%, 38.46%, 40.00%, 45.00%, 50.00%, 55.00%, 58.44%, or 60.00%, wherein the mass percentage refers to the percentage of the mass of the film-forming material relative to the total mass of the ambroxol oral soluble film composition (e.g., the ambroxol hydrochloride oral soluble film composition).

According to an embodiment of the present disclosure, preferably, the ambroxol oral soluble film composition (e.g., the ambroxol hydrochloride oral soluble film composition) described herein may further comprise one or more of a plasticizer, a flavoring agent, a colorant, and a filler. As an example, the ambroxol oral soluble film composition (e.g., the ambroxol hydrochloride oral soluble film composition) may or may not comprise the flavoring agent.

According to an embodiment of the present disclosure, the plasticizer refers to a substance for reducing the glass transition temperature of the film, increasing the plasticity and toughness, and improving the stretching rate, and may be selected from one or more of polyethylene glycol, glycerol, propylene glycol, silicone oil, polypropylene glycol, and hexanediol.

Preferably, the plasticizer is present at a mass percentage of 0 to 30.00%, preferably 0 to 25.00%, e.g., 1.00%, 2.00%, 2.60%, 3.00%, 4.00%, 5.00%, 6.00%, 6.06%, 7.00%, 8.00%, 8.67%, 9.00%, 10.00%, 10.15%, 10.99%, 15.00%, 20.00%, 24.90%, 25.00%, or 30.00%, wherein the mass percentage refers to the percentage of the mass of the plasticizer relative to the total mass of the ambroxol oral soluble film composition (e.g., the ambroxol hydrochloride oral soluble film composition).

According to an embodiment of the present disclosure, the flavoring agent refers to a substance for flavoring, and may be selected from one, two, or more of aspartame, sucralose, fructose, sucrose, stevioside, neotame, glycyrrhizin, an essence, a spice, saccharin, and saccharin sodium.

Preferably, the flavoring agent is present at a mass percentage of 0 to 50.00%, preferably 0 to 45.00%, e.g., 1.00%, 1.10%, 1.20%, 1.30%, 1.33%, 1.40%, 1.50%, 1.60%, 1.70%, 1.80%, 1.90%, 2.00%, 2.10%, 2.20%, 2.30%, 2.40%, 2.50%, 2.60%, 2.70%, 2.80%, 2.90%, 3.00%, 3.05%, 3.50%, 3.85%, 4.00%, 5.00%, 6.00%, 7.00%, 8.00%, 9.00%, 9.09%, 10.00%, 12.00%, 15.00%, 20.00%, 25.00%, 30.00%, 35.00%, 40.00%, 42.87%, 45.00%, or 50.00%, wherein the mass percentage refers to the percentage of the mass of the flavoring agent relative to the total mass of the ambroxol oral soluble film composition (e.g., the ambroxol hydrochloride oral soluble film composition).

According to an embodiment of the present disclosure, the colorant refers to a substance that is capable of improving the appearance and color of a formulation and can be used for identifying the concentration of the formulation, distinguishing the application method, and reducing the patient's aversion to the administration, and is selected from one, two, or more of titanium dioxide, a pigment, and a lake.

Preferably, the colorant is present at a mass percentage of 0 to 2.00%, e.g., 0, 0.02%, 0.10%, 0.15%, 0.20%, 0.25%, 0.30%, 0.41%, 0.50%, 0.60%, 0.70%, 0.80%, 0.90%, 1.00%, 1.10%, 1.20%, 1.30%, 1.40%, 1.50%, 1.60%, 1.65%, 1.70%, 1.80%, 1.90%, or 2.00%, wherein the mass percentage refers to the percentage of the mass of the colorant relative to the total mass of the ambroxol oral soluble film composition (e.g., the ambroxol hydrochloride oral soluble film composition).

According to an embodiment of the present disclosure, the filler refers to a solid substance that is added to a material to improve the properties of the material, or to solubilize and increase weight, and to reduce the cost of the material, and may be selected from one or more of sucrose, glucose, maltose, lactose, sorbitol, xylitol, maltitol, galactitol, erythritol, dextrin, and trehalose.

Preferably, the filler is present at a mass percentage of 0 to 20.00%, e.g., 0%, 1.00%, 5.00%, 10.00%, 15.00%, or 20.00%, wherein the mass percentage refers to the percentage of the mass of the filler relative to the total mass of the ambroxol oral soluble film composition (e.g., the ambroxol hydrochloride oral soluble film composition).

According to an embodiment of the present disclosure, on a mass percentage basis, the ambroxol oral soluble film composition (e.g., the ambroxol hydrochloride oral soluble film composition) is selected from the following formulas 1-1 to 1-5:
formula 1-1, comprising: 15% to 22% of ambroxol or the pharmaceutically acceptable salt thereof (e.g., ambroxol hydrochloride), 18% to 52% of polacrilin potassium, 18% to 35% of the film-forming material, 8% to 12% of the plasticizer, and 0.3% to 7% of the flavoring agent and/or the colorant;
formula 1-2, comprising: 15% to 22% of ambroxol or the pharmaceutically acceptable salt thereof (e.g., ambroxol hydrochloride), 18% to 46% of polacrilin potassium, 18% to 60% of the film-forming material, 2% to 22% of the plasticizer, and 0 to 0.05% of the flavoring agent and/or the colorant;
formula 1-3, comprising: 17% to 21% of ambroxol or the pharmaceutically acceptable salt thereof (e.g., ambroxol hydrochloride), 35% to 40% of polacrilin potassium, 34% to 41% of the film-forming material, and 2% to 6% of the flavoring agent and/or the colorant;
formula 1-4, comprising: 9% to 13% of ambroxol or the pharmaceutically acceptable salt thereof (e.g., ambroxol hydrochloride), 22% to 48% of sodium polystyrene sulfonate, 30% to 40% of the film-forming material, 5% to 25% of the plasticizer, and 0.2% to 10% of the flavoring agent and/or the colorant; and
formula 1-5, comprising: 18% to 24% of ambroxol or the pharmaceutically acceptable salt thereof (e.g., ambroxol hydrochloride), 20% to 24% of sodium polystyrene sulfonate, 13% to 15% of the film-forming material, and 40% to 45% of the flavoring agent.

According to an embodiment of the present disclosure, in formulas 1-1 to 1-5, the film-forming material is one, two, or more of xanthan gum, polyvinyl alcohol, hydroxypropyl methylcellulose, pullulan, and sodium alginate.

According to an embodiment of the present disclosure, in formulas 1-1 to 1-5, the plasticizer is glycerol.

According to an embodiment of the present disclosure, in formulas 1-1 to 1-5, the colorant is a lake.

The ambroxol hydrochloride oral soluble film composition described herein may be any one of the following formulas:
formula 2-1: 18% to 22% of ambroxol or the pharmaceutically acceptable salt thereof (e.g., ambroxol hydrochloride), 48% to 52% of polacrilin potassium, 14% to 32% of the film-forming material, 6% to 10% of the plasticizer, and 1% to 3% of the flavoring agent and/or the colorant, wherein the percentage refers to the percentage of the mass of the component relative to the total mass of the composition;
formula 2-2: 14% to 18% of ambroxol or the pharmaceutically acceptable salt thereof (e.g., ambroxol hydrochloride), 39% to 43% of polacrilin potassium, 25% to 29% of the film-forming material, 9% to 13% of the plasticizer, and 1.5% to 6% of the flavoring agent and/or the colorant, wherein the percentage refers to the percentage of the mass of the component relative to the total mass of the composition;
formula 2-3: 13% to 17% of ambroxol or the pharmaceutically acceptable salt thereof (e.g., ambroxol hydrochloride), 36% to 40% of polacrilin potassium, 28% to 32% of the film-forming material, 8% to 12% of the plasticizer, and 1% to 11% of the flavoring agent and/or the colorant, wherein the percentage refers to the percentage of the mass of the component relative to the total mass of the composition;
formula 2-4: 17% to 21% of ambroxol or the pharmaceutically acceptable salt thereof (e.g., ambroxol hydrochloride), 17% to 21% of polacrilin potassium, 58% to 52% of the film-forming material, and 0.5% to 4% of the plasticizer, wherein the percentage refers to the percentage of the mass of the component relative to the total mass of the composition;
formula 2-5: 17% to 21% of ambroxol or the pharmaceutically acceptable salt thereof (e.g., ambroxol hydrochloride), 36% to 40% of polacrilin potassium, 34% to 41% of the film-forming material, and 2% to 6% of the flavoring agent and/or the colorant, wherein the percentage refers to the percentage of the mass of the component relative to the total mass of the composition;
formula 2-6: 14% to 18% of ambroxol or the pharmaceutically acceptable salt thereof (e.g., ambroxol hydrochloride), 38% to 42% of polacrilin potassium, 30% to 34% of the film-forming material, 9% to 13% of the plasticizer, and 0 to 0.02% of the flavoring agent and/or the colorant, wherein the percentage refers to the percentage of the mass of the component relative to the total mass of the composition;
formula 2-7: 14% to 18% of ambroxol or the pharmaceutically acceptable salt thereof (e.g., ambroxol hydrochloride), 37% to 41% of polacrilin potassium, 29% to 33% of the film-forming material, 8% to 12% of the plasticizer, and 0 to 6% of the flavoring agent and/or the colorant, wherein the percentage refers to the percentage of the mass of the component relative to the total mass of the composition;
formula 2-8: 14% to 18% of ambroxol or the pharmaceutically acceptable salt thereof (e.g., ambroxol hydrochloride), 38% to 42% of polacrilin potassium, 30% to 34% of the film-forming material, 9% to 13% of the plasticizer, and 0 to 0.5% of the flavoring agent and/or the colorant, wherein the percentage refers to the percentage of the mass of the component relative to the total mass of the composition;
formula 2-9: 13% to 17% of ambroxol or the pharmaceutically acceptable salt thereof (e.g., ambroxol hydrochloride), 43% to 47% of polacrilin potassium, 18% to 22% of the film-forming material, and 18% to 22% of the plasticizer, wherein the percentage refers to the percentage of the mass of the component relative to the total mass of the composition;
formula 2-10: 7% to 11% of ambroxol or the pharmaceutically acceptable salt thereof (e.g., ambroxol hydrochloride), 44% to 48% of sodium polystyrene sulfonate, 26% to 34% of the film-forming material, 4% to 8% of the plasticizer, and 1% to 15% of the flavoring agent and/or the colorant, wherein the percentage refers to the percentage of the mass of the component relative to the total mass of the composition;
formula 2-11: 10% to 14% of ambroxol or the pharmaceutically acceptable salt thereof (e.g., ambroxol hydrochloride), 21% to 25% of sodium polystyrene sulfonate, 35% to 39% of the film-forming material, 23% to 27% of the plasticizer, and 0 to 0.5% of the flavoring agent and/or the colorant, wherein the percentage refers to the percentage of the mass of the component relative to the total mass of the composition; and
formula 2-12: 20% to 22% of ambroxol or the pharmaceutically acceptable salt thereof (e.g., ambroxol hydrochloride), 20% to 22% of sodium polystyrene sulfonate, 12% to 16% of the film-forming material, and 30% to 50% of the flavoring agent and/or the colorant, wherein the percentage refers to the percentage of the mass of the component relative to the total mass of the composition.

According to an embodiment of the present disclosure, in formulas 2-1 to 2-12, the film-forming material is one, two, or more of xanthan gum, polyvinyl alcohol, hydroxypropyl methylcellulose, pullulan, and sodium alginate.

According to an embodiment of the present disclosure, in formulas 2-1 to 2-12, the plasticizer is glycerol.

According to an embodiment of the present disclosure, in formulas 2-1 to 2-12, the colorant is a lake.

The ambroxol oral soluble film composition described herein may be any one of the following formulas:
formula 3-1: 18% to 22% of ambroxol or the pharmaceutically acceptable salt thereof (e.g., ambroxol hydrochloride), 48% to 52% of polacrilin potassium, 11% to 15% of xanthan gum, 4% to 8% of polyvinyl alcohol, 6% to 10% of glycerol, and 1% to 3% of aspartame, wherein the percentage refers to the percentage of the mass of the component relative to the total mass of the composition;
formula 3-2: 14% to 18% of ambroxol or the pharmaceutically acceptable salt thereof (e.g., ambroxol hydrochloride), 39% to 43% of polacrilin potassium, 25% to 29% of polyvinyl alcohol, 9% to 13% of glycerol, 0.5% to 1.5% of stevioside, 0.5% to 1.5% of the essence, and 0.5% to 3% of the lake, wherein the percentage refers to the percentage of the mass of the component relative to the total mass of the composition;
formula 3-3: 13% to 17% of ambroxol or the pharmaceutically acceptable salt thereof (e.g., ambroxol hydrochloride), 36% to 40% of polacrilin potassium, 28% to 32% of polyvinyl alcohol, 8% to 12% of glycerol, 1% to 5% of sucralose, 1% to 5% of the essence, and 0 to 0.05% of the lake, wherein the percentage refers to the percentage of the mass of the component relative to the total mass of the composition;
formula 3-4: 17% to 21% of ambroxol or the pharmaceutically acceptable salt thereof (e.g., ambroxol hydrochloride), 17% to 21% of polacrilin potassium, 58% to 52% of hydroxypropyl methylcellulose, and 0.5% to 4% of glycerol, wherein the percentage refers to the percentage of the mass of the component relative to the total mass of the composition;
formula 3-5: 17% to 21% of ambroxol or the pharmaceutically acceptable salt thereof (e.g., ambroxol hydrochloride), 36% to 40% of polacrilin potassium, 23% to 27% of polyvinyl alcohol, 11% to 14% of pullulan, and 2% to 6% of sucralose, wherein the percentage refers to the percentage of the mass of the component relative to the total mass of the composition;
formula 3-6: 14% to 18% of ambroxol or the pharmaceutically acceptable salt thereof (e.g., ambroxol hydrochloride), 38% to 42% of polacrilin potassium, 30% to 34% of polyvinyl alcohol, 9% to 13% of glycerol, and 0 to 0.02% of the lake, wherein the percentage refers to the percentage of the mass of the component relative to the total mass of the composition;
formula 3-7: 14% to 18% of ambroxol or the pharmaceutically acceptable salt thereof (e.g., ambroxol hydrochloride), 37% to 41% of polacrilin potassium, 29% to 33% of polyvinyl alcohol, 8% to 12% of glycerol, 1% to 5% of sucralose, and 0 to 0.05% of a lake, wherein the percentage refers to the percentage of the mass of the component relative to the total mass of the composition;
formula 3-8: 14% to 18% of ambroxol or the pharmaceutically acceptable salt thereof (e.g., ambroxol hydrochloride), 38% to 42% of polacrilin potassium, 30% to 34% of polyvinyl alcohol, 9% to 13% of glycerol, 0 to 0.1% of neotame, and 0 to 0.05% of the lake, wherein the percentage refers to the percentage of the mass of the component relative to the total mass of the composition;
formula 3-9: 13% to 17% of ambroxol or the pharmaceutically acceptable salt thereof (e.g., ambroxol hydrochloride), 43% to 47% of polacrilin potassium, 18% to 22% of xanthan gum, and 18% to 22% of glycerol, wherein the percentage refers to the percentage of the mass of the component relative to the total mass of the composition;
formula 3-10: 7% to 11% of ambroxol or the pharmaceutically acceptable salt thereof (e.g., ambroxol hydrochloride), 44% to 48% of sodium polystyrene sulfonate, 16% to 20% of sodium alginate, 10% to 14% of polyvinyl alcohol, 4% to 8% of glycerol, 1% to 5% of sucralose, 1% to 5% of stevioside, and 1% to 5% of the essence, wherein the percentage refers to the percentage of the mass of the component relative to the total mass of the composition;
formula 3-11: 10% to 14% of ambroxol or the pharmaceutically acceptable salt thereof (e.g., ambroxol hydrochloride), 21% to 25% of sodium polystyrene sulfonate, 35% to 39% of hydroxypropyl methylcellulose, 23% to 27% of glycerol, and 0 to 0.5% of the lake, wherein the percentage refers to the percentage of the mass of the component relative to the total mass of the composition; and
formula 3-12: 20% to 22% of ambroxol or the pharmaceutically acceptable salt thereof (e.g., ambroxol hydrochloride), 20% to 22% of sodium polystyrene sulfonate, 12% to 16% of xanthan gum, 12% to 16% of sucralose, 12% to 16% of stevioside, and 12% to 16% of the essence, wherein the percentage refers to the percentage of the mass of the component relative to the total mass of the composition.

The ambroxol hydrochloride oral soluble film composition described herein may be any one of the following formulas:
formula I: 20.00% of ambroxol hydrochloride, 50.00% of polacrilin potassium, 13.33% of xanthan gum, 6.67% of polyvinyl alcohol, 8.67% of glycerol, and 1.33% of aspartame, wherein the percentage refers to the percentage of the mass of the component relative to the total mass of the composition;
formula II: 16.48% of ambroxol hydrochloride, 41.21% of polacrilin potassium, 27.47% of polyvinyl alcohol, 10.99% of glycerol, 1.10% of stevioside, 1.10% of the essence, and 1.65% of the lake,
wherein the percentage refers to the percentage of the mass of the component relative to the total mass of the composition;
formula III: 15.23% of ambroxol hydrochloride, 38.06% of polacrilin potassium, 30.45% of polyvinyl alcohol, 10.15% of glycerol, 3.05% of sucralose, 3.05% of the essence, and 0.02% of the lake, wherein the percentage refers to the percentage of the mass of the component relative to the total mass of the composition;
formula IV: 19.48% of ambroxol hydrochloride, 19.48% of polacrilin potassium, 58.44% of hydroxypropyl methylcellulose, and 2.60% of glycerol, wherein the percentage refers to the percentage of the mass of the component relative to the total mass of the composition;
formula V: 19.23% of ambroxol hydrochloride, 38.46% of polacrilin potassium, 25.64% of polyvinyl alcohol, 12.82% of pullulan, and 3.85% of sucralose, wherein the percentage refers to the percentage of the mass of the component relative to the total mass of the composition;
formula VI: 16.21% of ambroxol hydrochloride, 40.53% of polacrilin potassium, 32.43% of polyvinyl alcohol, 10.81% of glycerol, and 0.02% of the lake, wherein the percentage refers to the percentage of the mass of the component relative to the total mass of the composition;
formula VII: 15.70% of ambroxol hydrochloride, 39.26% of polacrilin potassium, 31.41% of polyvinyl alcohol, 10.47% of glycerol, 3.14% of sucralose, and 0.02% of the lake, wherein the percentage refers to the percentage of the mass of the component relative to the total mass of the composition;
formula VIII: 16.16% of ambroxol hydrochloride, 40.40% of polacrilin potassium, 32.32% of polyvinyl alcohol, 10.77% of glycerol, 0.32% of neotame, and 0.02% of the lake, wherein the percentage refers to the percentage of the mass of the component relative to the total mass of the composition;
formula IX: 15.00% of ambroxol hydrochloride, 45.00% of polacrilin potassium, 20.00% of xanthan gum, and 20.00% of glycerol, wherein the percentage refers to the percentage of the mass of the component relative to the total mass of the composition;
formula X: 9.09% of ambroxol hydrochloride, 45.45% of sodium polystyrene sulfonate, 18.18% of sodium alginate, 12.12% of polyvinyl alcohol, 6.06% of glycerol, 3.03% of sucralose, 3.03% of stevioside, and 3.03% of the essence, wherein the percentage refers to the percentage of the mass of the component relative to the total mass of the composition;
formula XI: 12.45% of ambroxol hydrochloride, 24.90% of sodium polystyrene sulfonate, 37.34% of hydroxypropyl methylcellulose, 24.90% of glycerol, and 0.41% of the lake, wherein the percentage refers to the percentage of the mass of the component relative to the total mass of the composition;
formula XII: 21.43% of ambroxol hydrochloride, 21.43% of sodium polystyrene sulfonate, 14.29% of xanthan gum, 14.29% of sucralose, 14.29% of stevioside, and 14.29% of the essence, wherein the percentage refers to the percentage of the mass of the component relative to the total mass of the composition;
formula XIII: 15.22% of ambroxol hydrochloride, 38.06% of polacrilin potassium, 30.45% of polyvinyl alcohol, 10.15% of glycerol, 3.05% of sucralose, 3.05% of the essence, and 0.02% of the lake, wherein the percentage refers to the percentage of the mass of the component relative to the total mass of the composition;
formula XIV: 16.17% of ambroxol hydrochloride, 40.40% of polacrilin potassium, 32.32% of polyvinyl alcohol, 10.77% of glycerol, 0.32% of neotame, and 0.02% of the lake, wherein the percentage refers to the percentage of the mass of the component relative to the total mass of the composition;
formula XV: 9.10% of ambroxol hydrochloride, 45.45% of sodium polystyrene sulfonate, 18.18% of sodium alginate, 12.12% of polyvinyl alcohol, 6.06% of glycerol, 3.03% of sucralose, 3.03% of stevioside, and 3.03% of the essence, wherein the percentage refers to the percentage of the mass of the component relative to the total mass of the composition; and
formula XVI: 21.43% of ambroxol hydrochloride, 21.43% of sodium polystyrene sulfonate, 14.29% of xanthan gum, 14.29% of sucralose, 14.29% of stevioside, and 14.27% of the essence, wherein the percentage refers to the percentage of the mass of the component relative to the total mass of the composition.

According to an embodiment of the present disclosure, the sum of the mass percentages of the components in the ambroxol oral soluble film composition (e.g., the ambroxol hydrochloride oral soluble film composition) is 100%. However, it will be appreciated that there may be a ±0.2% rounding error due to the rounding of the values.

The present disclosure further provides a method for preparing the ambroxol oral soluble film composition (e.g., the ambroxol hydrochloride oral soluble film composition), comprising the following steps:
step 1: preparing an aqueous solution of the active drug;
step 2: adding the taste-masking agent into the aqueous solution obtained in step 1, and stirring to give a sample;
step 3: centrifuging the sample obtained in step 2, and discarding a part of the supernatant until the residual lower layer material is 10-15 times the mass of the active drug, so as to give a drug-loaded resin;
step 4: dissolving the film-forming material in water, mixing the solution with one or more of the flavoring agent, the plasticizer, the colorant, and the filler (if available), and stirring homogeneously to give a gelatinous solution; and
step 5: transferring the drug-loaded resin obtained in step 3 to the gelatinous solution obtained in step 4, uniformly stirring the mixture to give a uniform gelatinous solution, and after the completion of stirring, deaerating the gelatinous solution in vacuum, applying, drying, and cutting to give the ambroxol hydrochloride oral soluble film formulation.

According to an embodiment of the present disclosure, in step 1, the active drug solution is preferably prepared in the dark.

According to an embodiment of the present disclosure, in step 1, the water is preferably purified water.

According to an embodiment of the present disclosure, in step 2, the stirring is preferably performed in the dark.

According to an embodiment of the present disclosure, in step 2, the time of the stirring is preferably 8 h or longer.

According to an embodiment of the present disclosure, in step 3, the centrifugation is preferably performed in a centrifuge, wherein the centrifuge may be a benchtop centrifuge; the centrifuge may be a low-speed centrifuge.

According to an embodiment of the present disclosure, the ambroxol oral soluble film composition (e.g., the ambroxol hydrochloride oral soluble film composition) has a film thickness of 10 µm to 300 µm, e.g., 20 µm to 100 µm.

According to an embodiment of the present disclosure, the ambroxol oral soluble film composition (e.g., the ambroxol hydrochloride oral soluble film composition) can be completely disintegrated in 900 mL of simulated saliva at 37±1 °C within 120 s, e.g., completely disintegrated within 80 s.

The present disclosure further provides use of the ambroxol oral soluble film composition (e.g., the ambroxol hydrochloride oral soluble film composition) in preparing a medicament for preventing and/or treating an indication, e.g., allergy, targeted by ambroxol or the pharmaceutically acceptable thereof.

According to an embodiment of the present disclosure, the medicament is an expectorant or a mucolytic.

According to an embodiment of the present disclosure, the medicament is used in acute or chronic respiratory tract diseases accompanied by abnormal secretion in respiratory tracts, particularly for the expectorant treatment of chronic bronchitis, the treatment of neonatal respiratory distress syndrome, and the adjuvant treatment of a lung surgery.

The present disclosure further provides a method for preventing and/or treating a disease or a condition, comprising administering to a patient (e.g., human or other mammal) in need the ambroxol oral soluble film composition (e.g., the ambroxol hydrochloride oral soluble film composition).

According to an embodiment of the present disclosure, the disease or the condition is selected from allergy, excessive phlegm production, and acute or chronic respiratory tract diseases accompanied by abnormal secretion in respiratory tracts, particularly chronic bronchitis, neonatal respiratory distress syndrome, and postsurgical symptoms of a lung surgery.

The ambroxol oral soluble film composition, particularly the ambroxol hydrochloride oral soluble film composition of the present disclosure has a good dissolution speed and can be quickly disintegrated in the oral cavity without a gritty feeling after dissolution, thus possessing an improved taste and patient compliance. Moreover, the ambroxol oral soluble film composition, particularly the ambroxol hydrochloride oral soluble film composition of the present disclosure features uniform film appearance and good flexibility. In the preparation process of the film solution, no sedimentation occurs, and the content uniformity meets the requirements.

The reagents and starting materials used in the present disclosure are commercially available.

### Beneficial Effects

The ambroxol oral soluble film composition (e.g., the ambroxol hydrochloride oral soluble film composition) provided by the present disclosure has the advantages of a lower thickness, good taste, further improved stability, fast dissolution in the oral cavity without drinking water, and a high oral absorption speed, thus featuring good marketing prospects.

Moreover, the ambroxol oral soluble film composition (e.g., the ambroxol hydrochloride oral soluble film composition) of the present disclosure has good starting material availability, a simple process, ease to operate, a high drug load, and good drug content uniformity, and is suitable for industrial production.

**DETAILED DESCRIPTION**

The present disclosure is further illustrated by the following examples, which, however, are not intended to limit the present disclosure. Experimental procedures without specified conditions in the following examples were conducted in accordance with conventional procedures and conditions, or in accordance with product instructions.

### Examples 1-12

The percentage contents of the components in Examples 1-12, as shown in the following Table 1, are the mass percentages of the components in the dried (anhydrous) formulations on the mass basis of each component:

**Table 1**

| Example | | Example 1 | Example 2 | Example 3 | Example 4 | Example 5 | Example 6 |
|---|---|---|---|---|---|---|---|
| Active drug | Ambroxol hydrochloride | 15g (20.00%) | 15g (16.48%) | 15g (15.23%) | 15g (19.48%) | 15g (19.23%) | 15g (16.21%) |
| Taste-masking agent | Sodium polystyrene sulfonate | / | / | / | / | / | / |
| | Polacrilin potassium | 37.5g (50.00%) | 37.5g (41.21%) | 37.5g (38.06%) | 15g (19.48%) | 30g (38.46%) | 37.5g (40.53%) |
| Film-forming material | Xanthan gum | 10g (13.33%) | / | / | / | / | / |
| | Sodium alginate | / | / | / | / | / | / |
| | Hydroxypropyl methylcellulose | / | / | / | 15 (58.44%) | / | / |
| | Polyvinyl alcohol | 5g (6.67%) | 25g (27.47%) | 30g (30.45%) | / | 20g (25.64%) | 30g (32.43%) |
| | Pullulan | / | / | / | / | 10g (12.82%) | / |
| Plasticizer | Glycerol | 6.5g (8.67%) | 10g (10.99%) | 10g (10.15%) | 2g (2.60%) | / | 10g (10.81%) |
| Flavoring agent | Sucralose | / | / | 3g (3.05%) | / | 3g (3.85%) | / |
| | Stevioside | / | 1g (1.10%) | / | / | / | / |
| | Aspartame | 1g (1.33%) | / | / | / | / | / |
| | Neotame | / | / | / | / | / | / |
| | Essence | / | 1g (1.10%) | 3g (3.05%) | / | / | / |
| Colorant | Lake | / | 1.5g (1.65%) | 0.02g (0.02%) | / | / | 0.02g (0.02%) |
| Purified water* | | 120g | 130g | 170g | 140g | 140g | 170g |

| Example | | Example 7 | Example 8 | Example 9 | Example 10 | Example 11 | Example 12 |
|---|---|---|---|---|---|---|---|
| Active drug | Ambroxol hydrochloride | 15g (15.70%) | 15g (16.16%) | 15g (15.00%) | 15g (9.09%) | 15g (12.45%) | 15g (21.43%) |
| Taste-masking agent | Sodium polystyrene sulfonate | / | / | / | 75g (45.45%) | 30g (24.90%) | 15g (21.43%) |
| | Polacrilin potassium | 37.5g (39.26%) | 37.5g (40.40%) | 45g (45.00%) | / | / | / |
| Film-forming material | Xanthan gum | / | / | 20g (20.00%) | / | / | 10g (14.29%) |
| | Sodium alginate | / | / | / | 30g (18.18%) | / | / |
| | Hydroxypropyl methylcellulose | / | / | / | / | 45g (37.34%) | / |
| | Polyvinyl alcohol | 30g (31.41%) | 30g (32.32%) | / | 20g (12.12%) | / | / |
| | Pullulan | / | / | / | / | / | / |
| Plasticizer | Glycerol | 10g (10.47%) | 10g (10.77%) | 20g (20.00%) | 10g (6.06%) | 30g (24.90%) | / |
| Flavoring agent | Sucralose | 3g (3.14%) | / | / | 5g (3.03%) | / | 10g (14.29%) |
| | Stevioside | / | / | / | 5g (3.03%) | / | 10g (14.29%) |
| | Aspartame | / | / | / | / | / | / |
| | Neotame | / | 0.3g (0.32%) | / | / | / | / |
| | Essence | / | / | / | 5g (3.03%) | / | 10g (14.29%) |
| Colorant | Lake | 0.02g (0.02%) | 0.02g (0.02%) | / | / | 0.5g (0.41%) | / |
| Purified water* | | 170g | 170g | 140g | 200g | 170g | 100g |

| | | | | | | | |
|---|---|---|---|---|---|---|---|
| "*" denotes the removal of the substance during the process; "/" denotes no addition. | | | | | | | |

The method for preparing the formulations of the above embodiments comprises:
1) weighing and proportioning:
   taking proper amounts of starting materials and auxiliary materials according to the formula for later use;
2) resin drug-loading:
   A: adding the active drug into a certain amount of purified water in the dark, and completely dissolving the active drug;
   B: adding the taste-masking agent into the above solution, and keeping stirring for 8 h or longer in the dark;
   C: centrifuging the above sample in a low-speed, high-capacity benchtop centrifuge, discarding a part of the supernatant until the mass of lower residues was 10-15 times that of the active drug;
3) preparation of gelatinous solution:
   D: dissolving the film-forming material in a certain amount of purified water, adding the flavoring agent, the plasticizer and the colorant (if available), and uniformly stirring;
   E: transferring the lower residues in step 2) to the solution prepared in step 3), uniformly stirring to give a uniform gelatinous solution and after the completion of stirring, deaerating the gelatinous solution in vacuum; and
4) applying, drying, and cutting:
   G: applying the above gelatinous solution using a coating machine, drying, and then cutting into a suitable size to give the ambroxol hydrochloride oral soluble film formulation.

### Comparative Examples 1-3

Comparative Examples 1-3 were prepared according to the same method in Examples 3-5 disclosed in CN102846581A:

**Table 2**

| Comparative Example | | Comparative Example 1 | Comparative Example 2 | Comparative Example 3 |
|---|---|---|---|---|
| Active drug | Ambroxol hydrochloride | 15g (18.26%) | 15g (17.17%) | 15g (14.65%) |
| Taste-masking agent | Polacrilin potassium resin | 7.5g (9.13%) | 15g (17.17%) | 30g (29.31%) |
| Flavoring agent | Sucralose | 7.6g (9.25%) | 7.6g (8.70%) | 7.6g (7.42%) |
| Filler | Microcrystalline cellulose | 23.5g (28.60%) | 17.6g (20.15%) | 17.6g (17.19%) |
| Plasticizer | Glycerol | 5.28g (6.43%) | 8.82g (10.10%) | 8.82g (8.62%) |
| Film-forming material | Polyvinyl alcohol-polyethylene glycol copolymer | 14.7g (17.89%) | 14.7g (16.83%) | 14.7g (14.36%) |
| | Xanthan gum | 1.76g (2.14%) | 1.76g (2.01%) | 1.76g (1.72%) |
| | Sodium alginate | 0.88g (1.07%) | 0.88g (1.01%) | 0.88g (0.86%) |
| pH regulator | Citric acid | 0.06g (0.07%) | 0.12g (0.14%) | 0.12g (0.12%) |
| Pigment | | 0.0018g (0.00%) | 0.0018g (0.00%) | 0.0018g (0.00%) |
| Essence | | 5.88g (7.16%) | 5.88g (6.73%) | 5.88g (5.74%) |
| Purified water* | | 1066g | 1080g | 1080g |

| | | | | |
|---|---|---|---|---|
| "*" denotes the removal of the substance during the process. | | | | |

### Performance test

The ambroxol hydrochloride oral soluble films prepared according to the above Comparative Examples 1-3 and Examples 3-8 were separately evaluated in terms of mechanical strength, disintegration time, and related substances.

### Test Example 1

### Procedures for mechanical strength test of soluble films

The ambroxol hydrochloride oral soluble films were tested on a texture analyzer, and the calculated tensile strength, percent elongation, and folding endurance are shown in the following Table 3 (6 samples tested for each example):

**Table 3**

| Sample name | Thickness/mm | Width/mm | Cross-sectional area/mm² | Maximum force/gf | Tensile strength/MPa | Average tensile strength/MPa | Maximum force distance/mm | Sample height/mm | Percent elongation | Average percent elongation | Folding endurance |
|---|---|---|---|---|---|---|---|---|---|---|---|
| Example 3 | 0.25 | 20 | 5 | 1387.9 | 2.7 | 2.6 | 0.95 | 2.083 | 46% | 44% | Fracture after 4 foldings in half at 180° |
| | 0.25 | 20 | 5 | 1202.5 | 2.4 | | 1.025 | 2.083 | 49% | | |
| | 0.25 | 20 | 5 | 1612.2 | 3.2 | | 1.125 | 2.083 | 54% | | |
| | 0.23 | 20 | 4.6 | 1283.2 | 2.7 | | 0.65 | 2.083 | 31% | | |
| | 0.26 | 20 | 5.2 | 952.7 | 1.8 | | 0.925 | 2.108 | 44% | | |
| | 0.25 | 20 | 5 | 1423.4 | 2.8 | | 0.825 | 2.083 | 40% | | |
| Example 4 | 0.20 | 20 | 4 | 1389.7 | 3.4 | 3.5 | 0.675 | 2.083 | 32% | 40% | Fracture after 4 foldings in half at 180° |
| | 0.21 | 20 | 4.2 | 1483.2 | 3.5 | | 0.875 | 2.083 | 42% | | |
| | 0.20 | 20 | 4 | 1120.3 | 2.7 | | 0.85 | 2.083 | 41% | | |
| | 0.21 | 20 | 4.2 | 1598.9 | 3.7 | | 0.925 | 2.083 | 44% | | |
| | 0.20 | 20 | 4 | 1470.7 | 3.6 | | 0.9 | 2.083 | 43% | | |
| | 0.19 | 20 | 3.8 | 1528.8 | 3.9 | | 0.725 | 2.083 | 35% | | |
| Example 5 | 0.17 | 20 | 3.4 | 1165.5 | 3.4 | 3.4 | 0.95 | 2.083 | 46% | 45% | Fracture after 4 foldings in half at 180° |
| | 0.16 | 20 | 3.2 | 991.3 | 3.0 | | 1.05 | 2.108 | 50% | | |
| | 0.15 | 20 | 3 | 1067.2 | 3.5 | | 1 | 2.108 | 47% | | |
| | 0.16 | 20 | 3.2 | 979.1 | 3.0 | | 1 | 2.108 | 47% | | |
| | 0.15 | 20 | 3 | 1170.7 | 3.8 | | 0.775 | 2.083 | 37% | | |
| | 0.15 | 20 | 3 | 1145.8 | 3.7 | | 0.9 | 2.083 | 43% | | |
| Example 6 | 0.24 | 20 | 4.8 | 1456.8 | 3.0 | 3.1 | 0.8 | 2.108 | 38% | 46% | Fracture after 4 foldings in half at 180° |
| | 0.25 | 20 | 5 | 1560.4 | 3.1 | | 0.825 | 2.108 | 39% | | |
| | 0.25 | 20 | 5 | 1658.9 | 3.3 | | 0.964 | 2.108 | 46% | | |
| | 0.24 | 20 | 4.8 | 1694.2 | 3.5 | | 1.038 | 2.108 | 49% | | |
| | 0.24 | 20 | 4.8 | 1453.6 | 3.0 | | 1.214 | 2.108 | 58% | | |
| | 0.23 | 20 | 4.6 | 1346.8 | 2.9 | | 0.987 | 2.108 | 47% | | |
| Example 7 | 0.24 | 20 | 4.8 | 1462.1 | 3.0 | 3.1 | 0.915 | 2.108 | 43 | 42% | Fracture after 4 foldings in half at 180° |
| | 0.24 | 20 | 4.8 | 1521.1 | 3.1 | | 0.876 | 2.108 | 42 | | |
| | 0.23 | 20 | 4.6 | 1398.4 | 3.0 | | 0.842 | 2.108 | 40 | | |
| | 0.25 | 20 | 5 | 1647.8 | 3.2 | | 1.056 | 2.108 | 50 | | |
| | 0.23 | 20 | 4.6 | 1439.5 | 3.1 | | 0.835 | 2.108 | 40 | | |
| | 0.23 | 20 | 4.6 | 1498.2 | 3.2 | | 0.847 | 2.108 | 40 | | |
| Example 8 | 0.25 | 20 | 5 | 1612.5 | 3.2 | 3.2 | 1.013 | 2.083 | 49 | 48% | Fracture after 4 foldings in half at 180° |
| | 0.25 | 20 | 5 | 1587.2 | 3.1 | | 1.025 | 2.083 | 49 | | |
| | 0.25 | 20 | 5 | 1563.2 | 3.1 | | 1.089 | 2.083 | 57 | | |
| | 0.23 | 20 | 4.6 | 1498.5 | 3.2 | | 0.875 | 2.108 | 42 | | |
| | 0.25 | 20 | 5 | 1675.3 | 3.3 | | 1.017 | 2.083 | 49 | | |
| | 0.25 | 20 | 5 | 1624.9 | 3.2 | | 0.98 | 2.083 | 53 | | |
| Comparative Example 1 | 0.15 | 20 | 3 | 739.3 | 2.4 | 2.2 | 0.25 | 2.083 | 12% | 17% | Fracture after 2 foldings in half at 180° |
| | 0.17 | 20 | 3.4 | 791.2 | 2.3 | | 0.3 | 2.083 | 14% | | |
| | 0.16 | 20 | 3.2 | 889 | 2.7 | | 0.35 | 2.083 | 17% | | |
| | 0.16 | 20 | 3.2 | 568.3 | 1.7 | | 0.34 | 2.108 | 16% | | |
| | 0.16 | 20 | 3.2 | 694.6 | 2.1 | | 0.41 | 2.083 | 20% | | |
| | 0.16 | 20 | 3.2 | 550.8 | 1.7 | | 0.425 | 2.083 | 20% | | |
| Comparative Example 2 | 0.19 | 20 | 3.8 | 815.9 | 2.1 | 1.8 | 0.525 | 2.083 | 25% | 21% | Fracture after 2 foldings in half at 180° |
| | 0.18 | 20 | 3.6 | 746.3 | 2.0 | | 0.425 | 2.083 | 20% | | |
| | 0.18 | 20 | 3.6 | 608.2 | 1.7 | | 0.4 | 2.083 | 19% | | |
| | 0.18 | 20 | 3.6 | 712.1 | 1.9 | | 0.325 | 2.083 | 16% | | |
| | 0.19 | 20 | 3.8 | 615.1 | 1.6 | | 0.55 | 2.083 | 26% | | |
| | 0.17 | 20 | 3.4 | 582.6 | 1.7 | | 0.45 | 2.083 | 22% | | |
| Comparative Example 3 | 0.21 | 20 | 4.2 | 587.9 | 1.4 | 1.1 | 0.295 | 2.083 | 14% | 13% | Fracture after 2 foldings in half at 180° |
| | 0.20 | 20 | 4 | 602.5 | 1.5 | | 0.325 | 2.083 | 16% | | |
| | 0.21 | 20 | 4.2 | 312.2 | 0.7 | | 0.325 | 2.083 | 16% | | |
| | 0.22 | 20 | 4.4 | 483.2 | 1.1 | | 0.265 | 2.083 | 13% | | |
| | 0.22 | 20 | 4.4 | 452.7 | 1.0 | | 0.325 | 2.108 | 15% | | |
| | 0.22 | 20 | 4.4 | 423.4 | 0.9 | | 0.125 | 2.083 | 6% | | |

| | | | | | | | | | | | |
|---|---|---|---|---|---|---|---|---|---|---|---|
| Note: the "cross-sectional area" in the above table refers to the surface area of the oral soluble film. | | | | | | | | | | | |

The maximum force (gf) refers to a maximum force that the oral soluble film is capable of resisting after the yield stage. Tensile strength (MPa) = maximum force (gf) ÷ 102 ÷ cross-sectional area (mm2); percent elongation = maximum force distance (mm) ÷ sample height (mm) × 100%.

The average tensile strength refers to the average value of the data of the 6 groups.

The maximum force distance refers to the distance of the oral soluble film elongation under the maximum force.

The average percent elongation is the average value of the data of the 6 groups.

The folding endurance refers to the tolerance to folding in half at 180° in the oral soluble film.

The above results demonstrate that the ambroxol hydrochloride oral soluble films prepared according to Examples 3-8 had higher tensile strength and folding endurance than those of Comparative Examples 1-3, and are more suitable for commercial production, transportation, storage, and administration to a patient.

### Test Example 2

6 pieces of oral soluble film prepared in each one of Examples 1-12 and Comparative Examples 1-3 were separately determined for oral soluble film disintegration time. The procedures are as follows: 900 mL of a phosphate-buffered medium pH 6.8 (see General Chapter 8004, Chinese Pharmacopoeia, 2020 Edition for the preparation) was added into a 1000 mL beaker and heated to 37 ± 0.5 °C. The 6 pieces of oral soluble films were simultaneously placed in the beaker loaded on the disintegration tester, and the complete disintegration time of the 6 pieces of oral soluble films of each Example as well as Comparative Example was recorded, compared, and summarized in the following Table 4.

**Table 4**

| Example | Disintegration time (s) |
|---|---|
| Example 1 | 51 |
| Example 2 | 42 |
| Example 3 | 45 |
| Example 4 | 24 |
| Example 5 | 36 |
| Example 6 | 44 |
| Example 7 | 53 |
| Example 8 | 48 |
| Example 9 | 30 |
| Example 10 | 70 |
| Example 11 | 72 |
| Example 12 | 37 |
| Comparative Example 1 | 150 |
| Comparative Example 2 | 80 |
| Comparative Example 3 | 75 |

The results demonstrate that the ambroxol hydrochloride oral soluble films prepared according to Examples 1-12 were completely disintegrated within 120 s.

### Test Example 3

### Test of related substances of oral soluble films

The starting/auxiliary material compatibility study was designed according to the mass ratio of ambroxol hydrochloride to citric acid in Comparative Examples 1-3. After letting to stand at a high temperature of 60 °C for 30 days, the starting/auxiliary material compatibility and the related substance levels in Comparative Examples 1-3 and Examples 3-8 were determined.

The related substances were determined according to the method for high-performance liquid chromatography (General Chapter 0512, the Chinese Pharmacopoeia, Volume IV, 2020 Edition).

### Solvent: methanol

Test sample solution: two pieces (7.5 mg) or one piece (15 mg) of the oral soluble film (e.g., the samples prepared in the above examples or comparative examples) were placed in a 20-mL measuring flask; an appropriate amount of methanol was added; the mixture was ultrasonicated for 15 min to dissolve ambroxol hydrochloride and left to stand for cooling. An ambroxol hydrochloride solution at a concentration of about 0.75 mg/mL was prepared with methanol, and the supernatant was taken as the test sample solution.

Reference solution: an appropriate amount of the test sample solution was precisely measured and diluted using methanol to give a solution at a concentration of about 1.5 µg/mL.

Chromatographic conditions: octylsilane bonded silica was used as the filler (Agient ZORBAX SB-C8 4.6 mm × 150 mm, 3.5 µm); 10 mmol/L diammonium phosphate solution (adjusted to pH 7.0 using phosphoric acid)-acetonitrile (50:50) was used as the mobile phase; the detection wavelength was 248 nm, the flow rate was 0.8 mL/min; the column temperature was 30 °C, the sample tray temperature was 4 °C, the sample injection volume was 10 µL, and the operational time was 30 min. System suitability requirement: in the chromatogram of the test sample solution, the resolution between the main peak and the adjacent chromatographic peaks should meet the requirements. Determination method: the test sample solution and the reference solution were precisely measured and separately injected into the liquid chromatograph, and the chromatogram was recorded for 30 min.

**Table 5. Impurity correction factors and limits**

| Impurity | Relative retention time | Correction factor* | Limit |
|---|---|---|---|
| AXS-IM-A | 0.75 | 1.0 | 0.2% |
| AXS-IM-B | 0.82 | 1.0 | 0.5% |
| AXS-IM-C | 2.14 | 1.0 | 0.2% |
| AXS-IM-E | 1.46 | 1.0 | 0.2% |
| AXS-IM-I | 0.64 | 1.0 | 0.5% |
| Additional individual impurity | / | 1.0 | 0.2% |
| Total impurities | / | / | 1.0% |

**Table 6. Detection results for related substances**

| Sample | Condition | AXS-IM-I | AXS-IM-A | AXS-IM-B | AXS-IM-E | AXS-IM-C | Maximum unknown individual impurity/% | Total impurities/% |
|---|---|---|---|---|---|---|---|---|
| Example 3 | 0 days | Not detected | Not detected | Not detected | Not detected | Not detected | 0.11 | 0.11 |
| | Accelerated for 1 month | Not detected | Not detected | Not detected | Not detected | Not detected | 0.12 | 0.17 |
| Example 4 | 0 days | Not detected | Not detected | Not detected | Not detected | Not detected | Not detected | Not detected |
| | Accelerated for 1 month | Not detected | Not detected | Not detected | Not detected | Not detected | 0.07 | 0.10 |
| Example 5 | 0 days | Not detected | Not detected | Not detected | Not detected | Not detected | 0.06 | 0.06 |
| | Accelerated for 1 month | Not detected | Not detected | Not detected | Not detected | Not detected | 0.05 | 0.07 |
| Example 6 | 0 days | Not detected | Not detected | Not detected | Not detected | Not detected | 0.10 | 0.10 |
| | Accelerated for 1 month | Not detected | Not detected | Not detected | Not detected | Not detected | 0.11 | 0.12 |
| Example 7 | 0 days | 0.01 | Not detected | 0.02 | 0.01 | Not detected | 0.03 | 0.11 |
| | Accelerated for 1 month | Not detected | Not detected | 0.02 | 0.02 | Not detected | 0.03 | 0.13 |
| Example 8 | 0 days | Not detected | Not detected | 0.02 | 0.01 | Not detected | 0.10 | 0.10 |
| | Accelerated for 1 month | Not detected | Not detected | 0.03 | 0.01 | Not detected | 0.12 | 0.12 |
| Ambroxol hydrochloride: citric acid* | 0 days | Not detected | Not detected | Not detected | Not detected | Not detected | Not detected | Not detected |
| | Accelerated for 1 month | Not detected | Not detected | Not detected | Not detected | Not detected | 0.55 | 0.72 |
| | 60 °C, 30 days | Not detected | Not detected | Not detected | Not detected | Not detected | 0.72 | 1.03 |
| Comparative Example 1 | 0 days | Not detected | Not detected | 0.21 | Not detected | Not detected | 0.24 | 0.47 |
| | Accelerated for 1 month | Not detected | Not detected | 0.51 | Not detected | Not detected | 0.31 | 0.85 |
| Comparative Example 2 | 0 days | Not detected | Not detected | 0.17 | Not detected | Not detected | 0.18 | 0.40 |
| | Accelerated for 1 month | Not detected | Not detected | 0.44 | Not detected | Not detected | 0.22 | 0.72 |
| Comparative Example 3 | 0 days | Not detected | Not detected | 0.19 | Not detected | Not detected | 0.14 | 0.35 |
| | Accelerated for 1 month | Not detected | Not detected | 0.34 | Not detected | Not detected | 0.24 | 0.64 |

| | | | | | | | | |
|---|---|---|---|---|---|---|---|---|
| Note: * denotes a ratio in accordance with the guidelines without adding an additional auxiliary material. | | | | | | | | |

The results demonstrate that the oral soluble films prepared in Examples 3-6 of the present disclosure had good stability, while those prepared in Comparative Examples 1-3 had relatively high unknown impurity contents. The oral soluble films of the present application have better stability than those of Comparative Examples 1-3.

### Test Example 4

### Test of dissolution curve of oral soluble films

The ambroxol hydrochloride oral soluble films prepared in Examples 1, 3, and 6-8 were placed on a stainless steel mesh disk for dissolution curve detection according to sMethod Four (paddle over the disk method), General Chapter 0931 Dissolution and Drug Release Test, the Chinese Pharmacopoeia, Volume IV, 2020 Edition, using the stirring paddle in Method One and the dissolution cup of Method Two. The test conditions are as follows:
Instrument: Tianda Tianfa dissolution tester.
Medium: a hydrochloric acid solution at pH 1.2, an acetate buffer at pH 4.5, and a phosphate buffer at pH 6.8.

Test conditions: a medium volume of 900 mL, a temperature of 37 ± 0.5 °C, a stirring rotation speed of 50 rpm, and sampling time of 5 min, 10 min, 15 min, and 30 min.

The dissolution results and dissolution curve test results of two batches of ambroxol hydrochloride oral soluble films are shown in the following table:

**Table 7**

| **Dissolution curve test results of ambroxol hydrochloride oral soluble films (pH 1.2)** | | | | | | | |
|---|---|---|---|---|---|---|---|
| Example | Time/min | 0 | 5 | 10 | 15 | 30 | |
| Example 1 | Cumulative dissolution rate/% | 0 | 96 | 97 | 98 | 98 | |
| n=6 | RSD/% | 0 | 3.26 | 2.16 | 1.73 | 1.43 | |
| Example 3 | Cumulative dissolution rate/% | 0 | 101 | 103 | 102 | 102 | |
| n=6 | RSD/% | 0 | 2.68 | 2.29 | 2.75 | 2.44 | |
| Example 6 | Cumulative dissolution rate/% | 0 | 98 | 99 | 99 | 100 | |
| n=6 | RSD/% | 0 | 2.53 | 3.47 | 1.40 | 1.24 | |
| Example 7 | Cumulative dissolution rate/% | 0 | 100 | 101 | 101 | 102 | |
| n=6 | RSD/% | 0 | 2.10 | 1.95 | 1.04 | 1.41 | |
| Example 8 | Cumulative dissolution rate/% | 0 | 95 | 97 | 99 | 100 | |
| n=6 | RSD/% | 0 | 3.47 | 2.57 | 2.14 | 1.58 | |

| **Dissolution curve test results of ambroxol hydrochloride oral soluble films (pH 4.5)** | | | | | | | |
|---|---|---|---|---|---|---|---|
| **Example** | **Time/min** | **0** | **5** | **10** | **15** | **30** | **Similarity factor F2** |
| Example 1 | Cumulative dissolution rate/% | 0 | 73 | 79 | 82 | 86 | 52 |
| n=6 | RSD/% | 0 | 3.10 | 2.60 | 2.50 | 2.00 | |
| Example 3 | Cumulative dissolution rate/% | 0 | 86 | 89 | 91 | 94 | |
| n=6 | RSD/% | 0 | 3.11 | 3.33 | 3.5 | 3.94 | |
| Example 6 | Cumulative dissolution rate/% | 0 | 87 | 89 | 92 | 94 | / |
| n=6 | RSD/% | 0 | 3.41 | 2.53 | 2.14 | 1.08 | |
| Example 7 | Cumulative dissolution rate/% | 0 | 90 | 93 | 95 | 96 | |
| n=6 | RSD/% | 0 | 3.02 | 2.84 | 2.47 | 1.58 | |
| Example 8 | Cumulative dissolution rate/% | 0 | 84 | 88 | 93 | 95 | |
| n=6 | RSD/% | 0 | 3.15 | 3.01 | 2.58 | 2.17 | |

| **Dissolution curve test results of ambroxol hydrochloride oral soluble films (pH 6.8)** | | | | | | | |
|---|---|---|---|---|---|---|---|
| **Batch No.** | **Time/min** | **0** | **5** | **10** | **15** | **30** | |
| Example 1 | Cumulative dissolution rate/% | 0 | 82 | 87 | 89 | 92 | |
| n=6 | RSD/% | 0 | 1.73 | 2.73 | 3.71 | 3.26 | |
| Example 3 | Cumulative dissolution rate/% | 0 | 87 | 90 | 91 | 93 | |
| n=6 | RSD/% | 0 | 3.18 | 3.44 | 3.32 | 3.68 | |
| Example 6 | Cumulative dissolution rate/% | 0 | 87 | 91 | 93 | 95 | |
| n=6 | RSD/% | 0 | 2.59 | 2.14 | 1.57 | 1.02 | |
| Example 7 | Cumulative dissolution rate/% | 0 | 89 | 92 | 94 | 95 | |
| n=6 | RSD/% | 0 | 4.21 | 3.47 | 2.58 | 1.87 | |
| Example 8 | Cumulative dissolution rate/% | 0 | 86 | 89 | 93 | 94 | |
| n=6 | RSD/% | 0 | 3.58 | 3.04 | 2.47 | 2.17 | |

The results demonstrate that, in the hydrochloride acid solution at pH 1.2 and the phosphate buffer at pH 6.8, all of the ambroxol hydrochloride oral soluble films of the present disclosure had a cumulative dissolution rate of 85% or greater in 15 min; in the acetate buffer at pH 4.5, the formulation batches in Examples 3 and 6-8 had a cumulative dissolution rate of 85% or greater in 15 min and all of the formulation batches in Example 1 had a cumulative dissolution rate of 85% or greater in 30 min.

### Test Example 4

### Palatability evaluation of oral soluble films

The ambroxol hydrochloride oral soluble film batches were prepared according to Example 3 and the palatability evaluation of the ambroxol hydrochloride oral soluble film batches was performed on 6 randomly selected volunteers comprehensively using a bitterness grade evaluation method and a multi-factor investigation evaluation method with reference to the *Technical Instruction Principles of Pediatric Drug Palate Design and Evaluation (Draft for Solicitation of Comments)* and the literature (Li Pan et al. Application and development of volunteer sensory test in drug taste evaluation [J]. Chinese Pharmaceutical Journal, 2017, 52(22):1971-1975).

According to the quality attributes of the oral soluble film, palatability evaluation was performed in the aspects of appearance, bitterness, sweetness, tingly taste, oral sense of foreign matters, etc., where bitterness, tingly taste, and oral sense of foreign matters are main evaluation characteristics; appearance and sweetness are main bonus characteristics. The grading and scoring rules are as follows:

**Table 8. Palatability evaluation of oral soluble films**

| **Grade** | **Description of bitterness** | | **Description of tingly taste** | | **Description of sense of foreign matters** | | | **Score range** |
|---|---|---|---|---|---|---|---|---|
| **I** | Barely any bitterness | | Barely any tingly sensation | | Barely any sense of foreign matters | | | 8-10 |
| **II** | Mild bitterness | | Mild tingly sensation | | Mild sense of foreign matters | | | 6-8 |
| **III** | Acceptable bitterness | | Acceptable tingly sensation | | Acceptable sense of foreign matters | | | 4-6 |
| **IV** | Tolerable, strong bitterness | | Tolerable, strong tingly sensation | | Tolerable, strong sense of foreign matters | | | 2-4 |
| **V** | Intolerable bitterness | | Intolerable tingly sensation | | Intolerable sense of foreign matters | | | 0-2 |

| **Grade** | **Description of appearance** | | | | **Description of sweetness** | | | **Score range** |
|---|---|---|---|---|---|---|---|---|
| **I** | Pleasant color and suitable size | | | | Moderate sweetness | | | 2-3 |
| **II** | Good color and acceptable size | | | | Mildly excessive sweetness or mild tastelessness | | | 1-2 |
| **III** | Excessive deep color or excessive size | | | | Excessive sweetness or tastelessness | | | 0-1 |
| **Score** | **Description of palatability** | | | | | | | |
| **30-36** | Pleasant palatability | | | | | | | |
| **24-30** | Acceptable palatability | | | | | | | |
| **18-24** | Fair palatability | | | | | | | |
| **12-18** | Poor palatability | | | | | | | |
| **0-12** | Unpleasant palatability and no flavoring effect | | | | | | | |

| **Sample batch No.** | **Subject** | **Bitterness** | **Tingly flavor** | **Sense of foreign matters** | | **Appearance** | **Sweetness** | **Total score** |
|---|---|---|---|---|---|---|---|---|
| Example 3 | 001 | 8 | 8 | 4 | | 3 | 2 | 25 |
| | 002 | 9 | 8 | 5 | | 3 | 2 | 27 |
| | 003 | 8 | 8 | 6 | | 3 | 2 | 27 |
| | 004 | 8 | 9 | 6 | | 3 | 2 | 28 |
| | 005 | 8 | 8 | 6 | | 3 | 2 | 27 |
| | 006 | 7 | 8 | 6 | | 3 | 2 | 26 |

The results demonstrate that all of 6 volunteers had a final palatability score within the range of 24-30, indicating that the ambroxol hydrochloride oral soluble film prepared in the present disclosure has good palatability and good acceptance.

The test data described above suggest that the ambroxol hydrochloride oral soluble film provided by the present disclosure has the advantages of thin thickness, good taste, stable properties, fast dissolution in the oral cavity without drinking water, and high oral absorption speed.

The embodiments of the technical solutions of the present disclosure have been described above by way of example. It will be appreciated that the claimed scope of the present disclosure is not limited to the embodiments described above. Any modification, equivalent replacement, improvement, and the like made by those skilled in the art without departing from the spirit and principle of the present disclosure shall fall within the claimed scope of the present application.

## Claims

1. An ambroxol oral soluble film composition, comprising an active drug, a film-forming material, and a taste-masking agent, wherein the active drug comprises 2-amino-3,5-dibromo-N-(trans-4-hydroxycyclohexyl)benzylamine or a pharmaceutically acceptable salt thereof, e.g., 2-amino-3,5-dibromo-N-(trans-4-hydroxycyclohexyl)benzylamine hydrochloride of the following formula I: 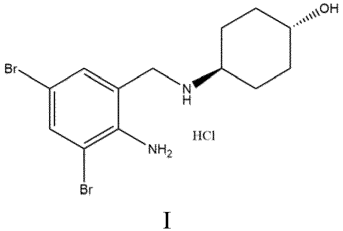
preferably, the ambroxol oral soluble film composition is an ambroxol hydrochloride oral soluble film composition;
preferably, the ambroxol oral soluble film composition (e.g., the ambroxol hydrochloride oral soluble film composition) does not comprise a pH regulator; for example, the pH regulator includes, but is not limited to a pharmaceutically acceptable acid for adjusting the pH.

2. The composition according to claim 1, wherein:
the active drug is present at a mass percentage of 1.00% to 40.00%, preferably 5.00% to 30.00%; the mass percentage refers to the percentage of the mass of the active drug relative to the total mass of the composition.

3. The composition according to claim 1 or 2, wherein the taste-masking agent is a cation exchange resin; the cation exchange resin is preferably selected from one, two, or three of sodium polystyrene sulfonate, polacrilin potassium, and polacrilin resin; preferably, the cation exchange resin has a particle size D90 of less than 200 µm;
and/or,
the taste-masking agent is present at a mass percentage of 10.00% to 70.00%, preferably 15.00% to 60.00%; the mass percentage refers to the percentage of the mass of the taste-masking agent relative to the total mass of the composition.

4. The composition according to any one of claims 1-3, wherein the film-forming material is selected from one or more of xanthan gum, guar gum, pectin, gelatin, shellac, gum arabic, starch, dextrin, agar, sodium alginate, zein, hydroxypropyl methylcellulose, hydroxymethylcellulose, hydroxyethylcellulose, hydroxypropylcellulose, polyvinyl alcohol, pullulan, polyvinylpyrrolidone, sodium alginate, polyethylene glycol, polyoxyethylene, acrylic acid copolymer, polylactic acid, and silicone rubber;
and/or,
the film-forming material is present at a mass percentage of 10.00% to 70.00%, preferably 15.00% to 60.00%; the mass percentage refers to the percentage of the mass of the film-forming material relative to the total mass of the composition.

5. The composition according to any one of claims 1-4, wherein the composition further comprises one or more of a plasticizer, a flavoring agent, a colorant, and a filler.

6. The composition according to claim 5, wherein:
the plasticizer is selected from one or more of polyethylene glycol, glycerol, propylene glycol, silicone oil, polypropylene glycol, and hexanediol;
and/or,
the flavoring agent is selected from one or more of aspartame, sucralose, fructose, sucrose, stevioside, neotame, glycyrrhizin, an essence, a spice, saccharin, and saccharin sodium;
and/or,
the colorant is selected from one or more of titanium dioxide, a pigment, and a lake;
and/or,
the filler is selected from one or more of sucrose, glucose, maltose, lactose, sorbitol, xylitol, maltitol, galactitol, erythritol, dextrin, and trehalose.

7. The composition according to claim 6, wherein:
the plasticizer is present at a mass percentage of 0 to 30.00%, preferably 0 to 25.00%; the mass percentage refers to the percentage of the mass of the plasticizer relative to the total mass of the composition;
and/or,
the flavoring agent is present at a mass percentage of 0 to 50.00%, preferably 0 to 45.00%; the mass percentage refers to the percentage of the mass of the flavoring agent relative to the total mass of the composition;
and/or,
the colorant is present at a mass percentage of 0 to 2.00%; the mass percentage refers to the percentage of the mass of the colorant relative to the total mass of the composition;
and/or,
the filler is present at a mass percentage of 0 to 20.00%; the mass percentage refers to the percentage of the mass of the filler relative to the total mass of the composition.

8. The composition according to any one of claims 1-7, wherein the composition is selected from any one of the following formulas:
formula 1-1, comprising: 15% to 22% of ambroxol or the pharmaceutically acceptable salt thereof (e.g., ambroxol hydrochloride), 18% to 52% of polacrilin potassium, 18% to 35% of the film-forming material, 8% to 12% of the plasticizer, and 0.3% to 7% of the flavoring agent and/or the colorant;
formula 1-2, comprising: 15% to 22% of ambroxol or the pharmaceutically acceptable salt thereof (e.g., ambroxol hydrochloride), 18% to 46% of polacrilin potassium, 18% to 60% of the film-forming material, 2% to 22% of the plasticizer, and 0 to 0.05% of the flavoring agent and/or the colorant;
formula 1-3, comprising: 17% to 21% of ambroxol or the pharmaceutically acceptable salt thereof (e.g., ambroxol hydrochloride), 35% to 40% of polacrilin potassium, 34% to 41% of the film-forming material, and 2% to 6% of the flavoring agent and/or the colorant;
formula 1-4, comprising: 9% to 13% of ambroxol or the pharmaceutically acceptable salt thereof (e.g., ambroxol hydrochloride), 22% to 48% of sodium polystyrene sulfonate, 30% to 40% of the film-forming material, 5% to 25% of the plasticizer, and 0.2% to 10% of the flavoring agent and/or the colorant; and
formula 1-5, comprising: 18% to 24% of ambroxol or the pharmaceutically acceptable salt thereof (e.g., ambroxol hydrochloride), 20% to 24% of sodium polystyrene sulfonate, 13% to 15% of the film-forming material, and 40% to 45% of the flavoring agent.

9. The composition according to any one of claims 1-8, wherein the composition has a thickness of 10 µm to 300 µm;
and/or,
the composition is completely disintegrated in 900 mL of simulated saliva at 37±0.5 °C within 120 s.

10. Use of the composition according to any one of claims 1-9 in preparing a medicament for treating and/or preventing allergy,
wherein preferably, the medicament is an expectorant or a mucolytic;
preferably, the medicament is used in acute or chronic respiratory tract diseases accompanied by abnormal secretion in respiratory tracts, particularly for the expectorant treatment of chronic bronchitis, the treatment of neonatal respiratory distress syndrome, and the adjuvant treatment of a lung surgery.
